(19) Europäisches Patentamt / European Patent Office / Office européen des brevets

(11) **EP 4 286 833 B1**

(12) **EUROPEAN PATENT SPECIFICATION**

(45) Date of publication and mention
of the grant of the patent:
**06.08.2025  Bulletin 2025/32**

(51) International Patent Classification (IPC):
**G01N 21/78** (2006.01)     **G01N 31/22** (2006.01)
**G01N 33/18** (2006.01)

(21) Application number: **22176715.5**

(22) Date of filing: **01.06.2022**

(52) Cooperative Patent Classification (CPC):
**G01N 21/78; G01N 31/22; G01N 33/182;**
G01N 2201/1211

(54) **METHOD OF DETERMINING PHOSPHATE CONCENTRATION  IN A WATER SAMPLE**

VERFAHREN ZUM BESTIMMEN DER PHOSPHATKONZENTRATION IN EINER WASSERPROBE

PROCÉDÉ DE DÉTERMINATION DE LA CONCENTRATION DE PHOSPHATE DANS UN ÉCHANTILLON D'EAU

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**06.12.2023  Bulletin 2023/49**

(73) Proprietor: **Hach Lange GmbH**
**14163 Berlin (DE)**

(72) Inventors:
• **RUDDE, Heinz**
**41836 Hückelhoven (DE)**
• **KUSSMANN, Michael**
**40476 Düsseldorf (DE)**
• **HAHN, Dr. Markus**
**47906 Kempen (DE)**
• **LENHARD, Markus**
**41751 Viersen (DE)**

(74) Representative: **terpatent PartGmbB**
**Burgunderstraße 29**
**40549 Düsseldorf (DE)**

(56) References cited:
• **MCGRAW ET AL: "Autonomous microfluidic system for phosphate detection", TALANTA, ELSEVIER, AMSTERDAM, NL, vol. 71, no. 3, 30 January 2007 (2007-01-30), pages 1180 - 1185, XP005864558, ISSN: 0039-9140, DOI: 10.1016/J.TALANTA.2006.06.011**
• **MA JIAN ET AL: "Underway analysis of nanomolar dissolved reactive phosphorus in oligotrophic seawater with automated on-line solid phase extraction and spectrophotometric system", ANALYTICA CHIMICA ACTA, ELSEVIER, AMSTERDAM, NL, vol. 950, 15 November 2016 (2016-11-15), pages 80 - 87, XP029826012, ISSN: 0003-2670, DOI: 10.1016/J.ACA.2016.11.029**
• **LIANG ET AL: "Flow injection analysis of nanomolar level orthophosphate in seawater with solid phase enrichment and colorimetric detection", MARINE CHEMISTRY, ELSEVIER SCIENCE B.V., AMSTERDAM, NL, vol. 103, no. 1-2, 12 December 2006 (2006-12-12), pages 122 - 130, XP005809623, ISSN: 0304-4203, DOI: 10.1016/J.MARCHEM.2006.06.013**

EP 4 286 833 B1

**Description**

The present invention relates to a method for determining phosphate in a water sample in an analyzer with a photometric reaction chamber which can be heated and cooled.

Attempts have been made for many years to reduce phosphate pollution in the environment, in particular from wastewaters.

According to the German Waste Water Ordinance, water treatment plants having a designed capacity for servicing more than 10,000 inhabitants must adhere to a discharge concentration of phosphate of not more than 2 mg/l. This value is 1 mg/l for a designed capacity for more than 100,000 inhabitants.

An exact determination of phosphate content is therefore necessary.

The molybdenum blue method and the molybdenum yellow method are among the currently most commonly used methods for measuring orthophosphate in a water system, in particular in industrial water. The molybdenum blue method is very sensitive and is suitable for measuring orthophosphate in low concentrations. However, in industrial water system, the orthophosphate concentration may sometimes reach as high as tens of ppm. When the orthophosphate concentration in water is high, the molybdenum blue method is easily saturated so that the water sample must be diluted before the measurement is performed. In contrast to the molybdenum blue method, the molybdenum yellow method has a wide range of measurement and is therefore more suitable for industrial water samples.

The documents MCGRAW ET AL: "Autonomous microfluidic system for phosphate detection", TALANTA, ELSEVIER, AMSTERDAM, NL, vol.71, no. 3, 30 January 2007 (2007-01-30), pages 1180-1185, ISSN:0039-9140, DOI: 10.1016/J. TALANTA 2006.06.011, MA JIAN ET AL: "Underway analysis of nanomolar dissolved reactive phosphorus in oligotrophic seawater with automated on-line solid phase extraction and spectrophotometric system", ANALYTICA CHIMICA ACTA, ELSEVIER, AMSTERDAM, NL, vol. 950, 15 November 2016 (2016-11-15), pages 80-87, ISSN:0003-2670, DOI: 10.1016/J.ACA.2016.11.029, and LIANG ET AL: "Flow injection analysis of nanomolar level orthophosphate in seawater with solid phase enrichment and colorimetic detection", MARINE CHEMISTRY, ELSEVIER SCIENCE B.V. AMSTERDAM, NL, vol. 103, no.1-2, 12 December 2006 (2006-12-12), pages 122-130, ISSN: 304-4203, DOI: 10.1016/J.MARCHEM.2006.06.013 disclose the molybdenum method in water sample applications.

The first step of the molybdenum yellow method is to acidify the water sample and to add a molybdate, where the orthophosphate and the molybdate react in the acidic aqueous solution to generate a solution that contains the phosphomolybdic acid (a heteropoly acid). A color development agent, such as vanadate, is then added to the aforesaid phosphomolybdic acid containing solution to develop a yellow color. The absorbance of the water sample is subsequently measured photometrically using a spectrometer, thereby optically determining the concentration of the orthophosphate contained in water.

DE 27 28 706 A1 describes a method for automatic phosphate determination using the molybdenum yellow method which uses as a reagent a solution which contains 25 g of ammonium heptamolybdate, 1 g of ammonium monovanadate, 56 ml of concentrated sulfuric acid, and 1 g of sodium chloride per liter. The molybdate and vanadate components form a yellow-colored complex together with phosphate. It is presumed that the following reaction takes place:

$$(NH_4)_6Mo_7O_{24} + NH_4VO_3 + PO_4^{3-} \rightarrow (NH_4)_3PO_4 \times NH_4VO_3 \times 16\ MoO_3$$

The equilibrium lies on the right-hand side of the equation in an acid medium (pH < 2). In this method, a wastewater sample is measured photometrically, the reagent is added, and after a delay time of from 4 to 5 minutes, a second measurement is carried out. The phosphate concentration can be determined from the yellow coloration with the aid of a standard solution. The intensity of the yellow color is here proportional to the phosphate concentration when the measurement solution is strongly acidic.

Variants of the above method are also known.

For example, WO 2016/133882 A1 describes a variant where the composition for measuring the concentration of orthophosphate in a water system is provided in solid form, in particular as a dried powder, in order to facilitate transportation, packaging and use. The composition for measuring the concentration of orthophosphate in a water system comprises:

(a) An acid in solid form;
(b) A water soluble molybdate; and
(c) A color development agent for developing the phosphomolybdic acid.

WO 2016/133882 A1 describes that the color development agent for developing the phosphomolybdic acid is a color development agent used in the molybdenum yellow method that develop a color for the phosphomolybdic acid formed from the reaction between the molybdate and the orthophosphate in the water system under the acidic conditions, for example, vanadates, for example, ammonium vanadate, sodium vanadate and potassium vanadate.

Another variant of molybdenum yellow method is described, for example, in US 4,544,639 A which describes a process for determining the amount of organic phosphonate present in an aqueous solution, comprising the steps of:

(a) Adding 1000 to 20,000 ppm of a strong oxidizing agent and 8 ppb to 5000 ppm silver ion to the aqueous solution;
(b) Allowing the organic phosphonate present in said solution to react to form orthophosphate;
(c) Precipitating any excess silver in said solution by the addition of 1000 to 50,000 ppm of a reducing agent or at least an equimolar amount of a halide, based on the silver ion added in Step (a), and removing any precipitate formed;
(d) Adding to said solution 5 to 30 percent, by volume, of an aqueous acid solution containing 500 to 2000 ppm of ions selected from the group consisting of molybdate ion and vanadate ion, and if a halide is used in Step (c), adding 1000 to 50,000 ppm of a reducing agent;
(e) Measuring the absorbance of said solution at 625 to 880 nanometers and determining the orthophosphate concentration from a calibration curve of orthophosphate concentration versus absorbance; and
(f) Subtracting any orthophosphate present in the original aqueous solution from the orthophosphate concentration determined in Step (e), to give the total organic phosphonate concentration.

US 2020/0340925 A1 describes a variant of the molybdenum yellow method which focuses on reliably determining small phosphate concentrations. US 2020/0340925 A1 thereby describes a method for determining phosphate in a water sample comprising:

(a) Providing a water sample;
(b) Adding an acid so that the concentration of the acid in the water sample is at least 0.5 wt.-%;
(c) Performing a first photometric measurement of the water sample solution;
(d) Adding a coloring component to the photometric water sample solution;
(e) Performing a second photometric measurement; and
(f) Calculating the phosphate concentration from the difference between the first photometric measurement and the second photometric measurement.

US 2020/0340925 A1 describes that the coloring reagent, i.e., a reagent based on molybdate and vanadate, is divided by firstly adding an acid before a first photometric measurement is carried out and then adding the coloring component to the same water sample solution and carrying out the second photometric measurement.

Each of DE 27 28 706 A1, WO 2016/133882 A1, US 4,544,639 A and US 2020/0340925 A1 disclose examples of the molybdenum yellow method.

A disadvantage of the molybdenum yellow method is that inaccuracies have been observed, in particular, when processing a wastewater sample and/or an industrial water sample having a low phosphate content at warm temperatures.

An object of the present invention is to provide an improvement of the molybdenum yellow method to overcome the above inaccuracies.

The present invention, defined in appended independent claim 1, provides a method for determining a phosphate concentration in a water sample in an analyzer with a photometric reaction chamber which can be heated and cooled. The method comprises: providing the water sample; determining a first phosphate concentration of the water sample via a molybdenum yellow method at a first temperature equal to or above 43°C; and if the first phosphate concentration falls within a specified range below or equal to 15 ppm, determining a corrected phosphate concentration of the same water sample via the molybdenum yellow method at a second temperature, below the first temperature.

In an embodiment of the present invention, if the specified range of the first phosphate concentration is < 5 ppm, the second temperature for the corrected phosphate concentration is preferably 30-35 °C, if the specified range of the first phosphate concentration is from 5-10 ppm, the second temperature for the corrected phosphate concentration is preferably 35-40 °C, and if the specified range of the first phosphate concentration is from 10-15 ppm, the second temperature for the corrected phosphate concentration is preferably 40-44 °C. According to the invention, as defined in appended claim 1, if the specified range of the first phosphate concentration falls below or equal to 15 ppm, the second temperature is below a first temperature of equal to or above 43 °C.

In an embodiment of the present invention, the first temperature is preferably from 43-47 °C, and most preferably at approximately 45 °C. The first temperature can, for example, be 43 °C, 44 °C, 45 °C, 46, °C 47 °C, 48 °C, 49, °C, 50 °C, 51 °C, 52 °C, 53 °C, 54 °C, 55 °C 56 °C, 57 °C, 58 °C, 59 °C or 60 °C.

In an example which does not fall within the scope of the present invention, the water sample is preferably divided into two water samples, for example, a first water sample and a second water sample. The determining of the first phosphate concentration is preferably performed with one of the two water samples, for example, with the first water sample, while the determining of the second phosphate concentration is performed with the other of the two water samples, for example, with the second water sample.

In an embodiment of the present invention, in accordance with appended claim 1, the determining of the first phosphate concentration and the determining of the second phosphate concentration is performed by adjusting the temperature of the water sample from the first temperature to the second temperature. A skilled person knows that the molybdenum yellow method will take a certain amount of time, for example, up to five minutes. Instead than running two samples, which would then up to 10 minutes, excluding any preparation time, the skilled person can instead use a single sample and perform the analysis with an analysis system where the single sample can be heated and/or cooled in situ. Analysis systems with heating and/or cooling systems have previously been described, for example, in US 4,004,708 A, US 4,619,530 A or in CN 104316479 A.

An aspect of the present invention therefore includes an analyzer having a photometric reaction chamber, for example with a flexible temperature range of 25-60 °C, which can be heated and/or cooled, for example between any temperature from 26-60 °C within a matter of seconds. This practically immediate temperature adjustment allows a photometric measurement to be conducted first at the first temperature, and almost immediately thereafter, at the second temperature, with the same sample. A skilled person could, merely as an example, perform a first analysis at 45 °C, and a second analysis at 30 °C. Any difference in the analysis result could thereby be the result of an interference. The use of two temperatures could therefore also be used by a skilled person to check and/or validate the analysis result obtained at another temperature. As stated above, any combination of temperatures between 25 and 60 °C is possible (provided that the starting temperature is not equal to the ending temperature), for example (in embodiments in accordance with the invention, the starting temperature must be equal to or above 43°C; the starting temperatures which are not equal to or above 43°C, and listed in the table below, are related to examples which do not fall within the scope of the invention defined by the appended claims):

| Starting Temperature | | | | Ending Temperature | | |
|---|---|---|---|---|---|---|
| 25 | 37 | 49 | | 25 | 37 | 49 |
| 26 | 38 | 50 | | 26 | 38 | 50 |
| 27 | 39 | 51 | | 27 | 39 | 51 |
| 28 | 40 | 52 | | 28 | 40 | 52 |
| 29 | 41 | 53 | | 29 | 41 | 53 |
| 30 | 42 | 54 | | 30 | 42 | 54 |
| 31 | 43 | 55 | Combined With | 31 | 43 | 55 |
| 32 | 44 | 56 | | 32 | 44 | 56 |
| 33 | 45 | 57 | | 33 | 45 | 57 |
| 34 | 46 | 58 | | 34 | 46 | 58 |
| 35 | 47 | 59 | | 35 | 47 | 59 |
| 36 | 48 | 60 | | 36 | 48 | 60 |

In an embodiment of the present invention, the water sample is preferably a wastewater sample or an industrial water sample. A skilled person knows that such samples are usually photometrically analyzed at approximately 45 °C.

The corrected phosphate concentration preferably mitigates an effect of an interference of the phosphate concentration at this temperature.

The interference is preferably a silicate interference. The term "silicate" is thereby understood to be any member of a family of anions consisting of silicon and oxygen, usually with the general formula $[SiO_{4-x}^{(4-2x)-}]n$ where $0 \leq x < 2$. The family includes orthosilicate $SiO_4^{4-}$ (x = 0), metasilicate $SiO_3^{2-}$ (x = 1), and pyrosilicate $Si_2O_7^{6-}$ (x = 0.5, n = 2). The name is also used for any salt of such anions, such as sodium metasilicate; or any ester containing the corresponding chemical group, such as tetramethyl orthosilicate. The term silicate is used to mean silicate minerals, ionic solids with silicate anions, as well as rock types that consist predominantly of such minerals. In that context, the term also includes the non-ionic compound silicon dioxide $SiO_2$ (silica, quartz), which would correspond to x = 2 in the general formula. The term also includes minerals where aluminum or other tetravalent atoms replace some of the silicon atoms, as in the aluminosilicates. The interference of the present invention is of course not limited to silicate, other examples of interference are also known to the skilled person, for example, Zr(IV) and Ti(IV), Ta(V) and Nb(V), W(VI).

A variant of the method for determining a phosphate concentration in a water sample, which does not fall within the scope of the present invention, includes providing the water sample, determining a first phosphate concentration of the water sample via a molybdenum yellow method or via a variant of the molybdenum yellow method at a temperature of from 40-50 °C, preferably from 43-47 °C, very preferably at approximately 45 °C, determining a silicate concentration of the water sample, and correcting the first phosphate concentration by applying a phosphate correction value so as to eliminate an interference effect of the silicate concentration.

A skilled person would know many methods of determining the silicate concentration of a water sample. One such method is described in ASTM D859-16 (2021)e1. Another method is the Spectroquant® Silicate Test 101813 of Merck KGaA which starts by pipetting 20 mL of sample solution into a plastic test vessel, after which 200 µL of a reagent (Si-1) is added. The solution is then mixed and then left to stand for 5 minutes. After the standing time, 200 µL a second reagent (Si-2) is added and the solution is mixed, then 1.00 mL of a third reagent (Si-3) is added. The solution is mixed once again, left to react for 5 minutes, and then measured in the photometer against a reagent blank prepared with Ultrapure water in an analogous manner. Another method is the "Silicomolybdate/Heteropoly Blue Method" of Hach Lange GmbH. The Silicomolybdate Method involves the reaction of molybdate ion with silica and phosphate under acid conditions to form a yellow color. Citric acid is added to destroy the phosphomolybdic acid complex (the yellow color formed due to phosphate), but not the silicomolybdic acid complex. For large amounts of silica, the remaining yellow color is intense enough to be read directly. For low concentrations, an amino-naphthol sulfonic acid reducing agent is used to convert the faint yellow color to a dark heteropoly blue species. The color formed is directly proportional to the amount of silica present in the original sample; a colorimetric measurement of this intensity provides an accurate means of determining the silica concentration. Some forms of silica (usually polymeric) will not react with ammonium molybdate and must be digested with sodium bicarbonate to be converted to a reactive form. Silicic acid reacts with water and hydrates as follows:

$$H_2SiO_3 + 3H_2O \rightarrow H_8SiO_6$$

This hydrated silicic acid reacts with molybdate in the presence of acids to form silicomolybdic acid.

$$H_8SiO_6 + 12(NH_4)_2MoO_4 + 12H_2SO_4 \rightarrow H_8[Si(Mo_2O_7)_6] + 12(NH_4)_2SO_4 + 12H_2O$$

This silicomolybdic acid is then reduced to a blue color (heteropoly species) by an amino naphthol sulfonic acid for low concentrations. The above examples are not intended to be limiting and a skilled person would have knowledge of additional methods.

In an example which does not fall within the scope of the present invention, the phosphate correction value is preferably,

a) from 0 to 25 ppm of silicate: (the determined ppm silicate) x 0.10/(25 ppm silicate),
b) from > 25 to 50 ppm of silicate: additionally to a), (the determined ppm silicate exceeding 25 ppm) x 0.07/(25 ppm silicate), and
c) from > 50 to 100 ppm of silicate: additionally to a) and b), (the determined ppm silicate exceeding 50 ppm) x 0.20/(50 ppm silicate).

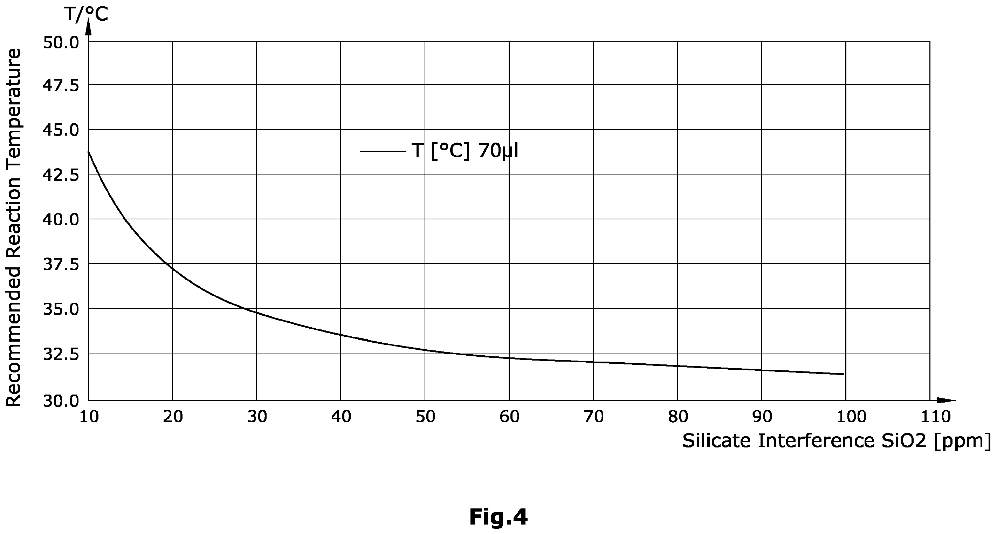

The above correction values are merely provided as an example. A skilled person can calculate narrower or broader ranges based on the water sample usually used. In an example which does not fall within the scope of the present invention, the phosphate correction value can also be provided by a look-up table and/or an assumption modeling which represents the interference effect of the silicate concentration at a specific temperature. An example of an assumption modeling is shown, for example, in Fig. 4. A skilled person would then deduct this interference effect from the first phosphate concentration based on the silicate concentration of the water sample determined, for example, in ppm silicate, and temperature at which the first phosphate concentration was determined. The corrected first phosphate concentration will then be obtained absent the interference effect of the silicate concentration.

A variant of the method for determining a phosphate concentration in a water sample in an example which does not fall within the scope of the present invention is to eliminate the interference effects of silicate by always determining the phosphate concentration at a temperature where no, or at least minimal, such interference effects occur. This variant preferably provides the water sample, establishes a temperature of the water sample of preferably 30-35 °C, and then determines the phosphate concentration of the water sample via the molybdenum yellow method or a variant thereof. The temperature of the water sample can, for example, even be lower than 30 °C, for example, from 10-30 °C. Preference is however given to a temperature of 30 °C, 31 °C, 32 °C, 33 °C and 35 °C, for example, 30 °C.

The present invention, defined by the appended claims, is described in greater detail below on the basis of embodiments and of the drawings in which:

Fig. 1 shows the optimizing of silicate interference at temperatures of 30 °C and 45 °C.

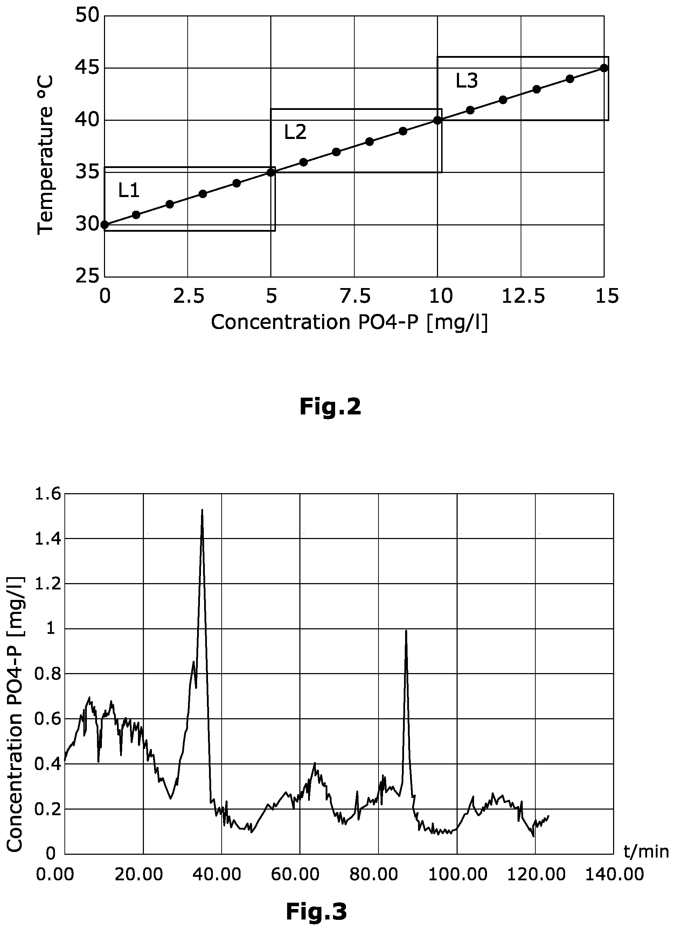

Fig. 2 shows an algorithm for forecasting a result of an increasing/decreasing phosphate concentration slope.

Fig. 3 shows a typically run of a wastewater plant in the range of < 5mg/l $PO_4$-P over time.

Fig. 4 shows an assumption modeling between reaction temperature and silicate interference.

The present invention, defined by the appended claims, is explained in greater detail below based on the following examples which are provided to show how the present invention can be practically applied in the real world. The examples are not intended to be limiting. The scope of the invention is defined and limited by the appended claims.

EXAMPLES

Example 1

In order to investigate the impact that silicate (here $SiO_2$) has on an orthophosphate measurement, a sample run was performed under standard conditions using the Phosphax sc Phosphat-Analysator from Hach Lange GmbH, 0.05-15 mg/l $PO_4$-P (55 $\mu$l reagent to 1 ml sample at 45 °C and less than 5 Minutes reaction time) with a silicate range of 0-100 mg/l $SiO_2$. It was thereby determined that the interference of silicate can result in up to 0.37 ppm phosphate equivalents as $PO_4$-P with 100 ppm silicate. Table 1 sets forth the data of samples run between 0-100 ppm $SiO_2$.

Table 1

| Sample | ppm $SiO_2$ | ppm PO4-P equivalent |
|---|---|---|
| 1 | 0 | 0.00 |
| 2 | 25 | 0.10 |
| 3 | 50 | 0.17 |
| 4 | 100 | 0.37 |

Example 2

A first sample run was repeated under optimized conditions using the Phosphax sc Phosphat-Analysator from Hach Lange GmbH, 0.05-15 mg/l $PO_4$-P (70 $\mu$l reagent to 1 ml sample at 45 °C and less than 5 Minutes reaction time) with a silicate range of 0-100 mg/l $SiO_2$. The only difference between Examples 1 and 2 was therefore that more reagent (70 $\mu$l instead of 55 $\mu$l) was used. It was thereby determined that the interference of silicate can result in up to 0.22 ppm phosphate equivalents as $PO_4$-P with 100 ppm silicate. Table 2 sets forth the data of samples run between 0-100 ppm $SiO_2$.

Table 2

| Sample | ppm $SiO_2$ | ppm PO4-P equivalent |
|---|---|---|
| 1 | 0 | 0.00 |
| 2 | 25 | 0.05 |
| 3 | 50 | 0.11 |
| 4 | 100 | 0.22 |

The interference impact could therefore be reduced by up to 40 % by manipulating the sample-to-reagent ratio.

Example 3

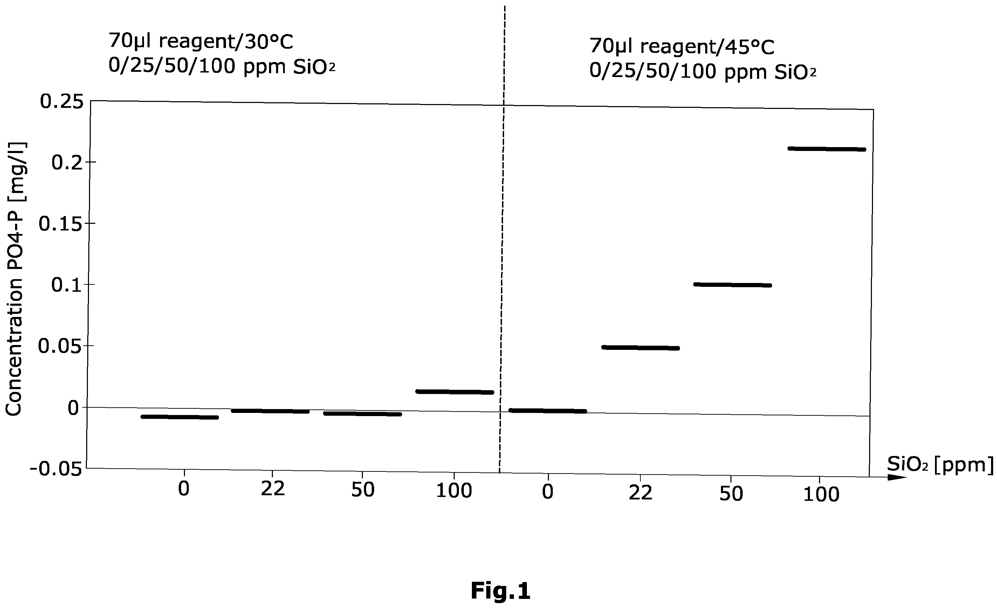

Example 2 was repeated at a temperature of 30 °C instead of 45 °C. It was thereby determined that the interference of silicate can be significantly reduced. Table 3 sets forth the data of samples run between 0-100 ppm $SiO_2$. A comparison of the results of Examples 2 and 3 are set forth in Fig. 1. Fig. 1 thereby clearly shows that the interference of silicate is significant at higher temperatures (i.e., 45 °C) compared to lower temperatures (i.e., 30 °C).

Table 3

| Sample | ppm SiO$_2$ | ppm PO4-P equivalent at 45 °C | ppm PO4-P equivalent at 30 °C |
|--------|-------------|-------------------------------|-------------------------------|
| 1 | 0 | 0.00 | -0.001 |
| 2 | 25 | 0.05 | 0.000 |
| 3 | 50 | 0.11 | 0.000 |
| 4 | 100 | 0.22 | 0.015 |

Examples 2 and 3 shows that the interference of silicate can be corrected based on a combination of temperature and sample-to-reagent ratio to approximately 93-100 %.

The above Examples demonstrate that varying only the ratio of sample-to-reagent will not in and of itself significantly minimize or entirely eliminate silicate interference. The above Examples also show, however, that changing the reaction temperature, for example, in the range of 30-60 °C, can significantly minimize or entirely eliminate silicate interference. While the present invention does not propose any conclusive underlying theory for this technical effect, it is believed that reaction kinetics may be influenced based on the Arrhenius equation in combination with the chosen concentration level. A practical application of this observation thereby involves dividing the measuring range into segmented reaction temperature areas or zones to thereby minimize or entirely eliminate silicate interference. For example, 50 mg/l of silicate showed an impact of 0.17 mg/l phosphate under standard conditions at 45 °C (see Example 1 above). A reduction of the temperature to 30 °C reduced this interference entirely (see Example 3 above). An analysis with high resolution down to 0.05 mg/l is therefore possible.

It is also possible to run an assumption modeling for various phosphate concentrations. Fig. 4 shows, for example, an assumption modeling between reaction temperature and silicate interference based on a reagent ratio of 70 µl to 1 ml sample. Fig. 4 thereby shows that the effect of silicate interference is reduced when the temperature is lowered. A skilled person can use such an assumption modeling to choose a reaction temperature with an expected silicate interference in order to minimize the impact of SiO$_2$.

**Claims**

1.   A method for determining a phosphate concentration in a water sample in an analyzer with a photometric reaction chamber which can be heated and cooled, the method comprising:

     providing the water sample;
     determining a first phosphate concentration of the water sample via a molybdenum yellow method at a first temperature equal to or above 43°C; and
     if the first phosphate concentration falls within a specified range below or equal to 15 ppm, determining a corrected phosphate concentration of the same water sample via the molybdenum yellow method at a second temperature, below the first temperature.

2.   The method as recited in claim 1, wherein,

     if the specified range of the first phosphate concentration is < 5 ppm, the second temperature for the corrected phosphate concentration is 30-35 °C,
     if the specified range of the first phosphate concentration is from 5-10 ppm, the second temperature for the corrected phosphate concentration is 35-40 °C, and
     if the specified range of the first phosphate concentration is from 10-15 ppm, the second temperature for the corrected phosphate concentration is 40-44 °C.

3.   The method as recited in claim 1 or 2, wherein the first temperature is from 43-47 °C, most preferably at approximately 45 °C.

4.   The method as recited in at least one of claims 1-3, wherein the determining of the first phosphate concentration and the determining of the second phosphate concentration is performed by adjusting the temperature of the water sample from the first temperature to the second temperature.

5.   The method as recited in at least one of claims 1-4, wherein the water sample is a wastewater sample or an industrial water sample.

6. The method as recited in at least one of claims 1-5, wherein the corrected phosphate concentration mitigates an effect of an interference of the a phosphate concentration.

7. The method as recited in claim 6, wherein the interference is a silicate interference.

**Patentansprüche**

1. Ein Verfahren zum Bestimmen einer Phosphatkonzentration in einer Wasserprobe in einem Analysator mit einer photometrischen Reaktionskammer, die erwärmt und gekühlt werden kann, wobei das Verfahren umfasst:

    Bereitstellen der Wasserprobe;
    Bestimmen einer ersten Phosphatkonzentration der Wasserprobe über eine Molybdängelb-Methode bei einer ersten Temperatur von gleich zu 43 °C oder darüber; und
    wenn die erste Phosphatkonzentration in einen bestimmten Bereich von unter oder gleich zu 15 ppm fällt, Bestimmen einer korrigierten Phosphatkonzentration derselben Wasserprobe über die Molybdängelb-Methode bei einer zweiten Temperatur unter der ersten Temperatur.

2. Das Verfahren gemäß Anspruch 1, wobei,

    wenn der bestimmte Bereich der ersten Phosphatkonzentration < 5 ppm beträgt, ist die zweite Temperatur für die korrigierte Phosphatkonzentration 30-35 °C,
    wenn der bestimmte Bereich der ersten Phosphatkonzentration von 5-10 ppm beträgt, ist die zweite Temperatur für die korrigierte Phosphatkonzentration 35-40 °C, und
    wenn der bestimmte Bereich der ersten Phosphatkonzentration 10-15 ppm beträgt, ist die zweite Temperatur für die korrigierte Phosphatkonzentration 40-44 °C.

3. Das Verfahren gemäß Anspruch 1 oder 2, wobei die erste Temperatur 43-47 °C beträgt, am bevorzugtesten ungefähr 45 °C.

4. Das Verfahren gemäß mindestens einem der Ansprüche 1-3, wobei das Bestimmen der ersten Phosphatkonzentration und das Bestimmen der zweiten Phosphatkonzentration durch Einstellen der Temperatur der Wasserprobe von der ersten Temperatur auf die zweite Temperatur durchgeführt wird.

5. Das Verfahren gemäß mindestens einem der Ansprüche 1-4, wobei die Wasserprobe eine Abwasserprobe oder eine industrielle Wasserprobe ist.

6. Das Verfahren gemäß mindestens einem der Ansprüche 1-5, wobei die korrigierte Phosphatkonzentration einen Effekt einer Interferenz der einen Phosphatkonzentration abschwächt.

7. Das Verfahren gemäß Anspruch 6, wobei die Interferenz eine Silikatinterferenz ist.

**Revendications**

1. Une méthode de détermination d'une concentration en phosphate dans un échantillon d'eau dans un analyseur avec une chambre de réaction photométrique qui peut être chauffée et refroidie, la méthode comprenant :

    la fourniture de l'échantillon d'eau ;
    la détermination d'une première concentration en phosphate de l'échantillon d'eau via une méthode de jaune de molybdène à une première température égale à ou au-dessus de 43 °C ; et
    si la première concentration en phosphate tombe dans une plage spécifiée au-dessous de ou égale à 15 ppm, la détermination d'une concentration en phosphate corrigée du même échantillon d'eau via la méthode de jaune de molybdène à une deuxième température, au-dessous de la première température.

2. La méthode de la revendication 1, dans laquelle,

si la plage spécifiée de la première concentration en phosphate est < 5 ppm, la deuxième température pour la concentration en phosphate corrigée est de 30 à 35 °C,

si la plage spécifiée de la première concentration en phosphate est de 5 à 10 ppm, la deuxième température pour la concentration en phosphate corrigée est de 35 à 40 °C, et

si la plage spécifiée de la première concentration en phosphate est de 10 à 15 ppm, la deuxième température pour la concentration en phosphate corrigée est de 40 à 44 °C.

3. La méthode de la revendication 1 ou 2, dans laquelle la première température est de 43 à 47 °C, idéalement d'environ 45 °C.

4. La méthode d'au moins une des revendications 1 à 3, dans laquelle la détermination de la première concentration en phosphate et la détermination de la deuxième concentration en phosphate sont effectuées en ajustant la température de l'échantillon d'eau de la première température à la deuxième température.

5. La méthode d'au moins une des revendications 1 à 4, dans laquelle l'échantillon d'eau est un échantillon d'eaux usées ou un échantillon d'eau industrielle.

6. La méthode d'au moins une des revendications 1 à 5, dans laquelle la concentration en phosphate corrigée atténue un effet d'une interférence de la concentration en phosphate.

7. La méthode de la revendication 6, dans laquelle l'interférence est une interférence de silicate.

Fig.1

**Fig.2**

**Fig.3**

**Fig.4**

EP 4 286 833 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DE 2728706 A1
- WO 2016133882 A1
- US 4544639 A
- US 20200340925 A1
- US 4004708 A
- US 4619530 A
- CN 104316479 A

**Non-patent literature cited in the description**

- Autonomous microfluidic system for phosphate detection. **MCGRAW et al.** TALANTA. ELSEVIER, 30 January 2007, vol. 71, 1180-1185
- Underway analysis of nanomolar dissolved reactive phosphorus in oligotrophic seawater with automated on-line solid phase extraction and spectrophotometric system. **MA JIAN et al.** ANALYTICA CHIMICA ACTA. ELSEVIER, 15 November 2016, vol. 950, 80-87
- Flow injection analysis of nanomolar level orthophosphate in seawater with solid phase enrichment and colorimetic detection. **LIANG et al.** MARINE CHEMISTRY. ELSEVIER, 12 December 2006, vol. 103, 122-130